# EUROPEAN PATENT APPLICATION

(11) **EP 4 134 083 A1**
(43) Date of publication of application: **15.02.2023**
(21) Application number: 22162914.0
(22) Date of filing: 18.03.2022
(51) Int. Cl.: A61K 31/69, A61K 38/05, A61K 9/08, A61K 47/10

(54) **PHARMACEUTICAL COMPOSITIONS OF BORTEZOMIB**

(30) Priority: 12.08.2021 IN 202121036483
(71) Applicant: Extrovis AG, 6340 Baar (CH)
(72) Inventor: CHILAKALA, Krishna Mohan, 500090 Hyderabad (IN); BEERAKA, Nagamalleswara Rao, 500090 Hyderabad (IN); KAMMA, Hanumantha Rao, 6340 Baar (CH); VACZI, Janos, 6403 Kuessnacht am Rigi (CH)
(74) Representative: Virnekäs, Bernhard

(57) **Abstract**

The present invention relates to a sterile, ready-to-use, aqueous composition of bortezomib that is stable over its shelf life.

## Description

The present invention relates to a stable composition of bortezomib comprising a therapeutically effective amount of bortezomib, a solvent and/or a cosolvent, wherein the composition is stable over its shelf life.

### BACKGROUND

Bortezomib is a modified dipeptidyl boronic acid. The chemical name for bortezomib, the monomeric boronic acid, is [(1R)-3-methyl-1-[[(2S)-1-oxo-3-phenyl-2-[(pyrazinylcarbonyl) amino]propyl]amino]butyl] boronic acid. Bortezomib has the following chemical structure:

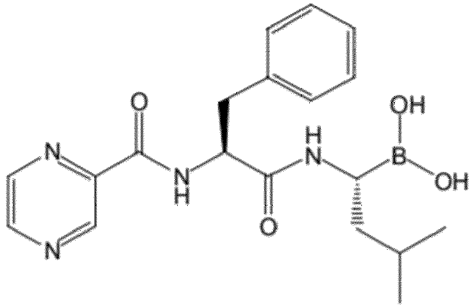

The molecular weight is 384.24. The molecular formula is C₁₉H₂₅BN₄O₄. The solubility of bortezomib, as the monomeric boronic acid, in water is 3.3 to 3.8 mg/mL in a pH range of 2 to 6.5.

Bortezomib is approved as a lyophilized powder for injection and is commercially available under the brand name VELCADE^{®}. Bortezomib is a proteasome inhibitor which is indicated for treatment of adult patients with multiple myeloma and mantle cell lymphoma. VELCADE^{®}. is available for intravenous injection or subcutaneous use. Each single-dose vial contains 3.5 mg of bortezomib as a sterile lyophilized powder. It also contains mannitol as the inactive ingredient. The product is provided as a mannitol boronic acid ester which, upon reconstitution, consists of the mannitol ester in equilibrium with its hydrolysis product, the monomeric boronic acid. The drug substance exists in its cyclic anhydride form as a trimeric boroxine.

VELCADE^{®}. contains no antimicrobial preservative. The reconstituted VELCADE^{®}. is to be administered within eight hours of preparation. When reconstituted as directed, VELCADE^{®} may be stored at 25°C (77°F) in the original vial and/or the syringe prior to administration, for up to eight hours. However, total storage time for the reconstituted material must not exceed eight hours when exposed to normal indoor lighting.

US5780454 discloses Bortezomib and its related compounds, and describes the synthetic process of preparation thereof.

Alkylboronic acids (including Bortezomib) are relatively difficult to obtain in the analytically pure form needed for preparation of medicines. Snyder et al., J. Am. Chem. Soc., 3611 (1958), explains that alkylboronic acid compounds readily form boroxines (anhydrides) under dehydrating conditions. Also, alkylboronic acids and their boroxines are often air-sensitive. Korcek et al., J. Chem. Soc., Perkin Trans. 2 242 (1972), shows that butylboronic acid is readily oxidized by air to generate 1-butanol and boric acid. These difficulties limit the pharmaceutical utility of boronic acid compounds, complicating the characterization of pharmaceutical agents comprising boronic acid compounds and limiting their shelf life.

US6713446 and US6958319 claim pharmaceutical compositions of boronic acid compounds prepared by lyophilization of an aqueous mixture comprising a boronic acid compound and a sugar, i.e. mannitol, wherein the lyophilized composition readily releases the boronic acid compound upon dissolution in aqueous media. The specifications of these patents disclose that the lyophilization with mannitol results in formation of the mannitol ester of bortezomib, which provides stability and allows storage at 2-8°C; bortezomib being unstable, requires storage below -20°C.

US8263578, US9061037, US9107821 and US9180093 describe liquid formulations of bortezomib comprising propylene glycol as a solvent, contributing to more than 50% of the formulation, and acetate buffer. The formulation has a pH of about 3. The use of propylene glycol in such high concentrations may result in adverse effects such as lactic acidosis, hyperosmolarity, hemolysis and renal toxicity, including tubular dysfunction and acute tubular necrosis.

WO2018164513A1 provides a bortezomib liquid formulation comprising at least one sugar alcohol selected from the group consisting of mannitol, erythritol, sorbitol, isomalt, maltitol, xylitol and trehalose, and a solvent formed by mixing propylene glycol and polar solvents including water. The pH range of the bortezomib liquid formulations is preferably 4.0 to 6.0.

The ratio of Bortezomib (or its salt) and sugar alcohol is 1:10 to 1:60. While this application discloses stable liquid compositions of bortezomib where the degradation rate of bortezomib is less than 2.5% at 25°C and 60% relative humidity, the disclosure is limited to the specific excipients disclosed and does not characterise the specific impurities and/or their limits. Further, para 71 of the specification states that, based on the stability results of examples 33-35, the use of acetate buffer is more advantageous than the use of water for injection. The working examples included in the specification, which include propylene glycol, mannitol, acetate buffer and/or water for injection (see examples 1, 2, 4-7, 20, 21, 30), are shown to undergo significant degradation within 21 days, and there is nothing in the specification to provide a teaching or suggestion to obtain bortezomib compositions that are stable over a shelf life of 12 months or more.

WO20160110870 provides stable ready-to-use injectable pharmaceutical formulation of bortezomib in a non-aqueous solvent system comprising solvents selected from the group comprising propylene glycol, one or more short chain alcohols (C1-C6), e.g. ethanol, dimethyl acetamide, N-methyl pyrrolidone, dimethyl sulphoxide, glycerol and mixtures thereof. The amount of non-aqueous solvents is at least 60%w/v.

US20170143622 discloses a stable liquid ready-to-use injectable formulation of bortezomib or pharmaceutically acceptable salts thereof, comprising a solvent system suitable for injection, wherein the solvent system comprises less than about 50% v/v of a non-aqueous solvent. The formulation also comprises other excipients like solubilizer, stabilizer, buffering agent, tonicity adjusting agent and pH adjusting agent.

US20170239335 relates to a stable, non-aqueous and ready-to-use injectable composition of bortezomib comprising a non-aqueous solvent system, polyol, pH adjusting agent and antioxidant.

WO2019097413 claims a stable non-aqueous pharmaceutical composition comprising bortezomib, cyclodextrin and propylene glycol.

US20180110822 relates to stable, ready-to-use liquid formulation of bortezomib comprising chelating agents such as DOTA, DTPA, EDTA; solvents like ethanol, glycerine, water; stabilizers such as mannitol, sorbitol, arginine, glycine and lysine; and anti-oxidants such as monothioglycerol.

The reconstitution of bortezomib lyophilized formulations is troublesome and there can be problems with chemical instability. Further, lyophilization makes the process more expensive and time consuming. Liquid compositions that contain non-aqueous solvents, and/or watermiscible solvents have their own limitations in terms of the quantity that can be used, the side effects arising out of their use, as well as the shelf life of the liquid composition. For example, Ever Pharma has an approved bortezomib solution product containing mannitol, sodium chloride, sodium hydroxide, hydrochloric acid and water for injection, which is stored at 2-8°C but has a shelf life of only 12 months (See https://mri.cts-mrp.eu/human/downloads/AT_H_1124_001_PAR.pdf)

Further, Wu et al (see "Degradation pathways of a peptide boronic acid derivative, 2-Pyz-(CO)-Phe-Leu-B(OH)(2)", Journal of Pharmaceutical Sciences, 2000, 89(6): 758-65), discusses possible degradation products of bortezomib under various conditions. The authors report that a polyethylene glycol (PEG) 300: Ethanol:Water (40:10:50) solvent system results in about 20% degradation at 25°C in 24 hours. The authors also report that use of ethanol and normal saline solution in a 2:98 ratio at pH 2.8 led to 20% degradation at 25°C in 1 month. In propylene glycol (PG): Ethanol:Water, the stability of bortezomib, while better, showed 20% degradation in 8 months when stored at 25° C. The disclosures of Wu et al, therefore, seem to teach that liquid solutions of bortezomib containing water and well-known, non-aqueous solvents, are subject to degradation and would pose difficulties in providing compositions with long shelf life.

Therefore, considering the issues disclosed above, a need exists for providing a stable aqueous composition of bortezomib that can be administered parenterally, and which is stable over its shelf life.

### DESCRIPTION

The present invention relates to a stable aqueous pharmaceutical composition of bortezomib for parenteral administration comprising
a. bortezomib or its pharmaceutically acceptable salt
b. one or more solvent(s)
c. one or more cosolvent(s)
d. one or more isotonicity agent(s)
e. optionally one or more stabilizer(s)
f. optionally a chelating agent
wherein the composition is a liquid that is ready-to-use and/or is ready-to-dilute for administration to a patient in need thereof, and is free of preservatives, antioxidants and buffers.

The present invention also provides stable aqueous pharmaceutical composition of bortezomib for parenteral administration comprising
a. bortezomib or its pharmaceutically acceptable salt
b. one or more solvent(s)
c. one or more cosolvent(s)
d. one or more isotonicity agent(s)
e. optionally one or more stabilizer(s)
f. optionally a chelating agent
g. optionally one or more preservative(s)
wherein the composition is provided in the form of a multidose vial and is ready-to-use or ready-to-dilute.

According to the present invention, the bortezomib is present in the said composition in an amount ranging from about 0.05% w/v to about 0.5% w/v. The amount of bortezomib used in the liquid composition of the present invention ranges from about 0.5mg/ml to about 5mg/ml. In preferred embodiments, the amount of bortezomib in the composition is 2.5mg/ml. In some other embodiments, the amount of bortezomib in the composition is 1mg/ml.

The present invention comprises liquid compositions of bortezomib or its pharmaceutically acceptable salt in an aqueous solvent system comprising one or more solvents and cosolvents. These may be selected from, but are not limited to, glycerine, ethanol, propylene glycol, water and mixtures thereof. Preferred solvents are ethanol, propylene glycol, water and mixtures thereof. The aqueous liquid compositions of the present invention are concentrates comprising bortezomib or its pharmaceutically acceptable salt that are either (1) ready-to-dilute, i.e. can be diluted and administered intravenously, or (2) ready-to-use, i.e. can be administered subcutaneously without any further dilution. As such, the term "ready-to-use" covers use of the compositions of the present invention that can be administered without further dilution, as well as those that need dilution for intravenous administration.

The stable aqueous compositions of the present invention may further comprise cosolvents in suitable amounts. The compositions of the present invention may include solvents and cosolvents, or mixtures thereof, in amount ranging from about 0.05% w/v to about 5% w/v.

According to the present invention, the composition may comprise tonicity adjusting agents. Tonicity adjusting agents decrease the hemolysis of blood cells and reduce pain and irritation at the injection site. The stable aqueous compositions of the present invention have osmolality ranging between about 280 mosm/kg and about 360 mosm/kg. Tonicity adjusting agents that can be included in the compositions of the present invention may be selected from mannitol, lactose, dextrose, glycerol and mixtures thereof, to provide the desired osmolality. A preferred tonicity adjusting agent is mannitol. The amount of the tonicity adjusting agent used is such that the desired osmolality is obtained. According to the present invention, the amount of tonicity adjusting agent may range from about 1% w/v to about 5% w/v.

According to the present invention, the pharmaceutical composition may comprise one or more stabilizers. A stabilizer, when present, may be selected from the group comprising arginine, alanine, glycine, methionine, lysine or proline, or a derivative or salt thereof, and mixtures thereof, in a concentration of from about 0.01% w/v to about 2% w/v. A preferred stabilizer may be arginine.

According to the present invention, the pharmaceutical composition may comprise a chelating agent. The chelating agent may be selected from DOTA (1, 4, 7, 10 tetraazacyclododecane -1, 4, 7, 10 - tetraacetic acid), EDTA (Ethylenediaminetetraacetic acid), ODDA (1, 4, 10, 13 - tetraoxa -7, 16 - diazacyclooctadecane -7), EGTA (ethylene glycol - bis (β - aminoethyl ether) - tetraacetic acid). The amount of chelating agent, when present, may be at least about 0.005%w/v.

According to the present invention, the composition may be provided in single dose vials or multidose vials that are free from preservatives, antioxidants and buffers, or may be provided in the form of multidose vials containing preservative, but which are free of antioxidants and buffers. The amount and type of preservative(s), when present, is selected from those conventionally used in parenteral compositions and well-known in the pharmaceutical art.

The fill volume for the vials containing the bortezomib compositions of the present invention may vary from about 1ml to about 10ml. Typically, a single dose of bortezomib is included in a volume of about 1.4ml, while multidose vials are available in a volume of 5ml. The stable, ready-to-use aqueous compositions of the present invention are typically filled in USP Type-I clear glass vials and are stoppered with inert rubber stoppers that are typically used in the pharmaceutical arts.

According to the present invention, the pH of the composition is in the range of about 4.5 to about 6.5. The pH is critical to the stability of the compositions of the present invention, and help in providing liquid compositions that are stable over the shelf life.

The present invention also provides a process for the preparation of sterile stable aqueous injectable compositions of bortezomib or its pharmaceutically acceptable salt. The processes are detailed in the examples included herein below. The compositions of the present invention are prepared under aseptic manufacturing conditions and filled in suitable vials, i.e. either in single dose vials or multidose vials. The headspace of the vials is then charged with nitrogen, thereby providing a nitrogen blanket above the aqueous composition of the invention, and ensuring that the dissolved oxygen in the aqueous compositions is not more than 2ppm.

The compositions of the present invention can be administered parenterally via an intravenous or a subcutaneous route. For administration by a subcutaneous route, the composition can be administered as is, and may not require further dilution. Typically, subcutaneous administration involves use at a concentration of 2.5mg/ml of bortezomib. For administration by an intravenous route, the composition will need to be diluted prior to administration. Typically, intravenous administration involves use at a concentration of 1mg/ml.

The sterile, ready-to-use, aqueous compositions of the present invention may be packaged in clear glass vials, followed by light resistant secondary packaging. The material of construction of the vials is such that stability and sterility of the bortezomib composition is not compromised over its shelf-life. The composition can be stored at a temperature of about 2°C to about 8°C. As previously described, the pharmaceutical compositions of the present invention are preconcentrates that are ready-to-dilute for intravenous administration, and are ready-to-use for subcutaneous administration. The preconcentrate or ready-to-dilute compositions of the present invention may be diluted with conventional diluents used for intravenous administration in the pharmaceutical art, such as dextrose, normal saline and the like. Upon dilution and/or the first opening of the vial, the composition is stable for about 28 days, when stored at a temperature of about 2°C to about 8°C when protected from light, and is stable for up to 7 days when stored in the original vial at about 25 degrees Celsius in normal indoor lighting conditions.

The pharmaceutical composition of the present invention is stable such that the bortezomib content after storage at (i) a temperature of about 2°C to about 8°C, and/or (ii) under accelerated storage conditions of 25°C and 60% relative humidity for six months, is not less than 90%. Preferably, the bortezomib content ranges between 90% and about 110%. Under the above described storage conditions, the maximum unspecified impurities in the pharmaceutical composition are not more than about 0.5% by weight of the composition. Further, the total impurities in the pharmaceutical composition, under the storage conditions described above, are not more than about 3.5% by weight of the composition.

The preferred specification for the sterile, ready-to-use, aqueous composition of the present invention, when stored at about 2°C to about 8°C, is as seen in Table 1 below. Such a composition has a shelf life of at least 12 months.

**Table 1**

| **Parameter** | **Specification** |
|---|---|
| pH | 4.5-6.5 |
| Osmolality | 280-360 mosm/kg |
| Assay (%) | NLT 90.0% to NMT 105.0% |
| Impurity C | NMT 0.5% |
| Impurity B | NMT 0.5% |
| Impurity I | NMT 0.5% |
| Impurity J | NMT 0.5% |
| Maximum unspecified/unknown impurities | NMT 0.5% |
| Total impurities | NMT 3.5% |

The names and structures of the known impurities are provided below -

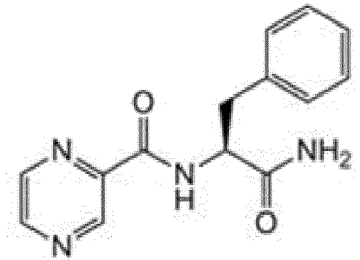

### Impurity C : (2S)-3-Phenyl-2-[(pyrazin-2-ylcarbonyl)amino]propanamide ; or N-(1-Amino-1-oxo-3-phenylpropan-2-yl)pyrazine-2-carboxamide

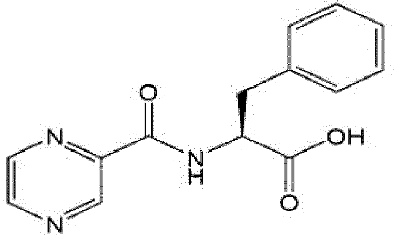

### Impurity B : (2S)-3-Phenyl-2-[(pyrazin-2-ylcarbonyl) amino] propanoic acid; or N-(2-Pyrazinyl carbonyl)-L-phenylalanine ;(S)-3-Phenyl-2-(pyrazine-2-carboxamido) propanoic acid

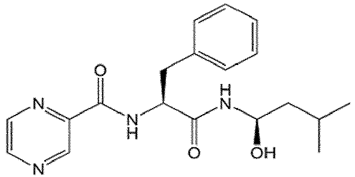

### Impurity I : N-[(S)-1-{[(S)-1-Hydroxy-3-methylbutyl]amino}-1-oxo-3-phenylpropan-2-yl]pyrazine-2-carboxamide

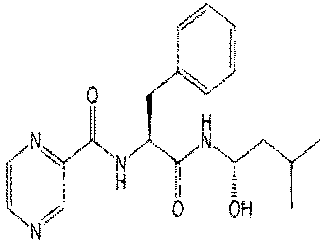

### Impurity J : N-[(S)-1-{[(R)-1-Hydroxy-3-methylbutyl]amino}-1-oxo-3-phenylpropan-2-yl]pyrazine-2-carboxamide

The term "shelf life", as used herein, refers to the amount of time the pharmaceutical composition may be stored without loss of potency and/or performance profile, i.e. compositions that stay within the specification defined in Table 1 above, upon storage at about 2°C to about 8°C, and/or at about 25°C and 60% relative humidity. The stable compositions provided herein are designed to have shelf life of at least 12 months, 24 months or 36 months. Preferred embodiments of the present invention have a shelf life of about 24 months.

### Definitions

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

Unless the context indicates otherwise, it is specifically intended that the various features of the invention described herein can be used in any combination.

Moreover, the present invention also contemplates that in some embodiments of the invention, any feature or combination of features set forth herein can be excluded or omitted.

All publications, patent applications, patents, and other references mentioned herein are incorporated by reference herein in their entirety for all purposes.

As used herein, "a," "an," or "the" can mean one or more than one.

Also, as used herein, "and/or" refers to and encompasses any and all possible combinations of one or more of the associated listed items, as well as the lack of combinations when interpreted in the alternative ("or").

As used herein, the term "about," as used herein when referring to a measurable value such as an amount of a compound or agent of this invention, dose, time, temperature, and the like, is meant to encompass variations of ± 10% of the specified amount.

"Ready to dilute" when used in connection with a bortezomib composition refers to a composition that includes bortezomib in dissolved or solubilized form, and may be used as such, or upon further dilution with intravenous diluents. The terms "composition" and "solution" may be used interchangeably.

As used herein, and unless otherwise specified, the term "stable" refers to physical and chemical stability of the composition.

The present invention is further illustrated by reference to the following examples which is for illustrative purposes only, and does not limit the scope of the invention in any manner.

### Example 1

| **Ingredients** | **Quantity per mL** | **Quantity in %** |
|---|---|---|
| Bortezomib | 2.5mg | 0.25 |
| Ethanol | 30mg | 3 |
| Mannitol | 25mg | 2.5 |
| Water for Injection | qs to 1mL | qs to 100 |
| pH 5.3 | | |

Bortezomib was dispensed and transferred to a vessel containing ethanol, and mixed and heated to 50-55°C until dissolved completely. Water for Injection was purged with nitrogen to obtain less than 2ppm of dissolved oxygen. About 90% of the purged water for injection was added into a container and heated to 50°C - 60°C. Mannitol was added and mixed until dissolved completely. The bortezomib solution was added slowly to the mannitol solution by continuous stirring at 50°C - 60°C. It was then cooled to room temperature and the volume was made up to batch size with water for injection previously purged with nitrogen.

The composition was subjected to stability studies, the results of which are recorded in Table 2 below -

**Table 2**

| **Condition** | **Initial** | **2-8°C** | **25°C±2°C/60±5% RH** | | | **40°C±2°C/ 75±5% RH** | **60°C** |
|---|---|---|---|---|---|---|---|
| **Parameter** | | **45days** | **12days** | **45days** | **60days** | **10days** | **1day** |
| Description | CCS | CCS | CCS | CCS | CCS | CCS | CCS |
| pH | 5.79 | NT | NT | NT | NT | NT | NT |
| Assay (%) | 100.83 | 100.01 | 101.9 | 99.06 | 97.68 | 99.36 | 97.25 |
| **Related substances by HPLC** | **%w/w** | | | | | | |
| Unknown Impurity @ RRT 0.20 | ND | ND | ND | ND | 0.03 | ND | ND |
| Impurity C | 0.01 | 0.01 | 0.01 | 0.04 | 0.08 | 0.12 | |
| Impurity B | 0.03 | 0.10 | 0.12 | 0.47 | 0.68 | 0.63 | 0.22 |
| Unknown Impurity @ RRT 1.24 | ND | 0.04 | 0.07 | 0.09 | 0.19 | 0.27 | 0.19 |
| Unknown Impurity @ RRT 1.50 | 0.12 | 0.12 | 0.20 | 0.15 | 0.19 | 0.17 | 0.15 |
| Unknown Impurity @ RRT 1.91 | 0.03 | 0.06 | 0.11 | 0.16 | 0.22 | 0.15 | 0.07 |
| Total Impurities | 0.43 | 0.48 | 0.63 | 1.14 | 1.55 | 1.40 | 0.84 |

| | | | | | | | |
|---|---|---|---|---|---|---|---|
| Note: NT : Not tested; W: Week; CCS: Clear Colourless solution; RRT : Relative Retention Time | | | | | | | |

The composition of Example 1 was subjected to further stability testing, the results of which are recorded in Tables 3 and 4 below.

**Table 3**

| **Condition** | **Initial** | **2-8°C** | | | | **25°C±2°C/60±5% RH** | | | | **40°C±2°C/ 75+5% RH** | |
|---|---|---|---|---|---|---|---|---|---|---|---|
| **Parameter** | | **1M** | **2M** | **3M** | **6M** | **1M** | **2M** | **3M** | **6M** | **1W** | **2W** |
| Description | CCS | CCS | CCS | CCS | CCS | CCS | CCS | CCS | CCS | CCS | CCS |
| pH | 5.24 | 5.33 | 5.55 | 5.60 | 5.66 | 5.54 | 5.54 | 5.63 | 5.70 | NT | NT |
| Assay (%) | 102.7 | 102.1 | 102.1 | 101.4 | 102.8 | 100.8 | 100.9 | 100.7 | 100.0 | 102.8 | 100.1 |

**Table 4**

| **Related substances by HPLC (%w/w)** | | | | | | | | | | | |
|---|---|---|---|---|---|---|---|---|---|---|---|
| | **Initial** | **2-8°C** | | | | **25°C±2°C/60±5% RH** | | | | **40°C±2°C/ 75+5% RH** | |
| | | **1M** | **2M** | **3M** | **6M** | **1M** | **2M** | **3M** | **6M** | **1W** | **2W** |
| Unknown Impurity @ RRT 0.24 | 0.01 | ND | ND | 0.00 | ND | 0.00 | ND | 0.02 | 0.09 | 0.01 | 0.01 |
| Impurity C | 0.01 | 0.01 | 0.01 | 0.01 | 0.02 | 0.03 | 0.05 | 0.07 | 0.13 | 0.06 | 0.12 |
| Impurity B | 0.04 | 0.05 | 0.06 | 0.11 | 0.14 | 0.32 | 0.60 | 0.87 | 1.68 | 0.48 | 1.02 |
| Unknown Impurity @ RRT 1.24 | 0.08 | 0.06 | 0.06 | 0.07 | 0.07 | 0.14 | 0.19 | 0.25 | 0.39 | 0.13 | 0.21 |
| Impurity I | 0.12 | 0.08 | 0.10 | 0.07 | 0.08 | 0.06 | 0.06 | 0.07 | 0.09 | 0.08 | 0.07 |
| Impurity J | 0.05 | 0.04 | 0.06 | 0.07 | 0.09 | 0.07 | 0.08 | 0.11 | 0.15 | 0.08 | 0.11 |
| Maximum unspecified impurities | 0.03 | 0.04 | 0.03 | 0.4 | 0.04 | 0.03 | 0.04 | 0.04 | 0.06 | 0.04 | 0.03 |
| Total Impurities | 0.46 | 0.41 | 0.43 | 0.50 | 0.55 | 0.74 | 1.16 | 1.61 | 2.79 | 1.02 | 1.74 |

### Example 2

| **Ingredients** | **Quantity per mL** | **Quantity in %** |
|---|---|---|
| Bortezomib | 2.5mg | 0.25 |
| Ethanol | 30mg | 3 |
| Mannitol | 25mg | 2.5 |
| Arginine | 0.23mg | 0.023 |
| Water for Injection | Qs to 1mL | QS to 100 |
| pH 6.6 | | |

The composition was obtained by process similar to that in Example 1 above. The results of the stability testing conducted for the composition are recorded in Table 5 below -

**Table 5 :**

| **Condition** | **Initial** | **2-8°C** | **2-8°C** | **25°C±2°C/ 60±5% RH** | | **40°C±2°C/ 75±5% RH** | **60°C** |
|---|---|---|---|---|---|---|---|
| **Parameter** | | **2W** | **1M** | **2W** | **1M** | **2W** | **ID** |
| Description | CCS | CCS | CCS | CCS | CCS | CCS | CCS |
| pH | 6.61 | NT | NT | NT | NT | NT | NT |
| Osmolality | NT | 715 | NT | 704 | NT | 705 | NT |
| Assay (%) | 100.8 | 98.3 | 99.5 | 100. 1 | 97.5 | 94.66 | 100.5 |
| **Related substances by HPLC** | **%w/w** | | | | | | |
| Unknown Impurity @ RRT 0.18 | ND | 0.01 | 0.06 | 0.44 | 0.81 | 0.39 | ND |
| Impurity C @ RRT 0.4 | 0.01 | 0.04 | 0.12 | 0.10 | 0.3 | 0.88 | 0.13 |
| Impurity B @ RRT 0.69 | 0.03 | 0.20 | 0.39 | 0.08 | 0.88 | 2.51 | 0.43 |
| Unknown Impurity @ RRT 1.24 | 0.08 | 0.16 | 0.25 | 0.30 | 0.52 | 1.97 | 0.42 |
| Unknown Impurity @ RRT 1.50 | 0.08 | 0.11 | 0.11 | 0.24 | 0.33 | 0.18 | 0.13 |
| Unknown Impurity @ RRT 1.91 | 0.01 | 0.03 | 0.03 | 0.07 | 0.12 | 0.08 | 0.03 |
| Total Impurities | 0.31 | 0.69 | 1.13 | 1.39 | 2.42 | 6.25 | 1.28 |

| | | | | | | | |
|---|---|---|---|---|---|---|---|
| Note: NT - Not tested; CCS: Clear Colourless solution; RRT : Relative Retention Time | | | | | | | |

The composition of Example 2 was subjected to further stability testing, the results of which are recorded in Tables 6 and 7 below -

**Table 6**

| **Condition** | **Initial** | **2-8°C** | | | |
|---|---|---|---|---|---|
| **Parameter** | | **1M** | **2M** | **3M** | **6M** |
| Description | CCS | CCS | CCS | CCS | CCS |
| pH | 6.61 | NT | NT | 6.43 | 6.47 |
| Osmolality | NT | NT | NT | 651 | 709 |
| Assay (%) | 100.8 | 99.5 | 99.5 | 98.9 | 100.0 |

**Table 7**

| **Related substances by HPLC (%w/w)** | | | | | |
|---|---|---|---|---|---|
| | **Initial** | **2-8°C** | | | |
| | | **1M** | **2M** | **3M** | **6M** |
| Unknown Impurity @ RRT 0.24 | ND | 0.06 | ND | ND | ND |
| Impurity C | 0.01 | 0.12 | 0.04 | 0.04 | 0.07 |
| Impurity B | 0.03 | 0.39 | 0.23 | 0.25 | 0.39 |
| Unknown Impurity @ RRT 1.24 | 0.08 | 0.25 | 0.14 | 0.22 | 0.31 |
| Impurity I | 0.08 | 0.11 | 0.12 | 0.11 | 0.12 |
| Impurity J | 0.01 | 0.03 | 0.03 | 0.03 | 0.04 |
| Maximum unspecified impurities | 0.06 | 0.04 | 0.05 | 0.04 | 0.06 |
| Total Impurities | 0.31 | 1.13 | 0.73 | 0.79 | 1.17 |

### Example 3

| **Ingredients** | **Quantity per mL** | **Quantity in %** |
|---|---|---|
| Bortezomib | 2.5mg | 0.25 |
| Ethanol | 30mg | 3 |
| Mannitol | 25mg | 2.5 |
| Arginine | 0.23mg | 0.023 |
| DOTA | 0.2mg | 0.02 |
| Water for Injection | qs to 1mL | qs to 100 |
| pH 5.77 | | |

Bortezomib was dispensed and transferred to a vessel containing ethanol, and the same was mixed and heated to 50 -55°C, until completely dissolved. Water for Injection was purged with nitrogen to obtain less than 2ppm of dissolved oxygen, and heated to 50°C - 60°C. Mannitol was added to the water for injection and mixed until dissolved completely. The bortezomib solution was added slowly to the mannitol solution by continuous stirring at 50°C - 60°C. Arginine and DOTA were added to the above mixture, and stirred for another 60mins at 50°C-60°C, until a solution was obtained. The solution was then cooled to room temperature and the volume was made up with water for injection previously purged with nitrogen.

The testing results for composition of Example 3 are shown in Table 8 below -

**Table 8**

| **Condition** | **Initial** | **60°C** |
|---|---|---|
| **Parameter** | | **1 day** |
| Description | CCS | CCS |
| pH | 5.77 | NT |
| Assay (%) | 99.34 | 95.93 |
| **Related substances by HPLC (%w/w)** | | |
| Unknown Impurity @ RRT 0.24 | ND | 0.05 |
| Impurity C | 0.01 | 0.12 |
| Impurity B | 0.01 | 0.47 |
| Unknown Impurity @ RRT 1.24 | 0.04 | 0.31 |
| Impurity I | 0.06 | 0.15 |
| Impurity J | 0.02 | 0.01 |
| Maximum unspecified impurities | 0.05 | 0.04 |
| Total Impurities | 0.27 | 1.41 |

### Example 4

| **Ingredients** | **Quantity per mL** | **Quantity per vial** | **Quantity (%w/v)** |
|---|---|---|---|
| Bortezomib | 2.5 mg | 3.5 mg | 0.25 % |
| Ethanol | 1.0 mg | 1.4 mg | 0.1 % |
| Propylene glycol | 9.0 mg | 12.6 mg | 0.9 % |
| Mannitol | 25 mg | 35 mg | 2.5 % |
| Water for Injection | qs to 1mL | qs to 1.4mL | qs to 100% |

Ethanol and propylene glycol were dispensed and transferred to a manufacturing vessel-1 and stirred to obtain a uniform solution. The solution was heated to 40-60°C. The required quantity of bortezomib was added into the above manufacturing vessel-1 and stirred until a clear solution was obtained by maintaining the temperature at 40-60°C. Water for injection was transferred to manufacturing vessel-2. Mannitol was added to vessel-2 and stirred until a clear solution was obtained. The solution was heated to 40-60°C. The bortezomib solution from compounding vessel-1 was slowly added to compounding vessel-2 and stirred until a clear solution was obtained by maintaining the temperature at 40-60°C. Finally the volume was made up to 100% using the remaining water for injection. The solution was then cooled to room temperature (20-25°C) and filtered using a 0.2 micron sterilizing grade filter. The solution was then filled in vials (Fill Volume: 1.4mL), stoppered and sealed. The final product was stored under refrigerated conditions.

### Example 5

The composition of Example 4 above was subjected to stability studies, the results of which are shown in Table 9 and Table 10 below -

**Table 9 :**

| **Condition** | **Initial** | **2-8°C** | | **25°C±2°C/ 60±5% RH** | | **40°C±2°C/ 75±5% RH** | | **60°C** |
|---|---|---|---|---|---|---|---|---|
| **Parameter** | | **1W** | **2W** | **1W** | **2W** | **1W** | **2W** | **1 day** |
| Description | CCS | CCS | CCS | CCS | CCS | CCS | CCS | CCS |
| pH | 5.20 | NT | NT | NT | NT | NT | NT | NT |
| Osmolality | 302 | 287 | NT | 286 | NT | 287 | NT | NT |
| Assay (%) | 98.11 | 96.7 | 97.64 | 96.5 | 96.5 | 95.7 | 95.9 | 104 |

**Table 10 :**

| **Related substances by HPLC (%w/w)** | | | | | | | | |
|---|---|---|---|---|---|---|---|---|
| | **Initial** | **2-8°C** | | **25°C±2°C/ 60±5% RH** | | **40°C±2°C/ 75±5% RH** | | **60°C** |
| | | **1W** | **2W** | **1W** | **2W** | **1W** | **2W** | **1 day** |
| Unknown Impurity @ RRT 0.20 | ND | 0.04 | 0.03 | 0.25 | 0.09 | 0.13 | 0.06 | BDL |
| Impurity C @ 0.4 | 0.08 | 0.09 | 0.08 | 0.10 | 0.09 | 0.14 | 0.18 | 0.07 |
| Impurity B @ 0.69 | 0.33 | 0.39 | 0.36 | 0.28 | 0.45 | 0.80 | 1.27 | 0.44 |
| Unknown Impurity @ RRT 1.24 | 0.06 | 0.14 | 0.10 | 0.12 | 0.12 | 0.23 | 0.24 | 0.20 |
| Unknown Impurity @ RRT 1.50 | 0.10 | 0.10 | 0.1 | 0.10 | 0.1 | 0.10 | 0.08 | 0.06 |
| Unknown Impurity @ RRT 1.91 | 0.14 | 0.15 | 0.15 | 0.15 | 0.16 | 0.16 | 0.14 | 0.09 |
| Total Impurities | 0.83 | 1.05 | 0.99 | 1.14 | 1.18 | 1.7 | 2.14 | 1.03 |

| | | | | | | | | |
|---|---|---|---|---|---|---|---|---|
| Note - W : Week; ND : Not detected; NT : Not tested; RRT : Relative Retention Time; BDL : below detection limit | | | | | | | | |

### Example 6

The composition of Example 4 above was subjected to additional stability testing, the results of which are shown in Table 11 and Table 12 below -

**Table 11 :**

| **Condition** | **Initia I** | **2-8°C** | | | | **25°C/60% relative humidity** | | | |
|---|---|---|---|---|---|---|---|---|---|
| **Parameter** | | **1M** | **2M** | **3M** | **6M** | **1M** | **2M** | **3M** | **6M** |
| Description | CCS | CCS | CCS | CCS | CCS | CCS | CCS | CCS | CCS |
| pH | 5.20 | NT | 5.36 | 5.58 | 5.41 | NT | 5.38 | 5.40 | 5.22 |
| Osmolality | 302 | NT | 296 | 293 | 290 | NT | 301 | 309 | 285 |
| Assay (%) | 98.1 | 96.4 | 96.0 | 96.3 | 95.3 | 96.2 | 95.2 | 95.8 | 94.1 |

| | | | | | | | | | |
|---|---|---|---|---|---|---|---|---|---|
| Note - M: Month; NT : Not tested; RRT : Relative Retention Time | | | | | | | | | |

**Table 12 :**

| | **Related substances by HPLC (%w/w)** | | | | | | | | | |
|---|---|---|---|---|---|---|---|---|---|---|
| | | **Initial** | **2-8°C** | | | | **25°C/60% relative humidity** | | | |
| | | | **1M** | **2M** | **3M** | **6M** | **1M** | **2M** | **3M** | **6M** |
| Unknown Impurity @ RRT 0.24 | | ND | 0.04 | 0.03 | 0.02 | 0.03 | 0.61 | 0.67 | 1.01 | 1.55 |
| Impurity C | | 0.08 | 0.09 | 0.07 | 0.08 | 0.08 | 0.11 | 0.11 | 0.13 | 0.14 |
| Impurity B | | 0.33 | 0.37 | 0.32 | 0.37 | 0.42 | 0.22 | 0.20 | 0.14 | 0.33 |
| Impurity I | | 0.10 | 0.1 | 0.08 | 0.10 | 0.09 | 0.10 | 0.08 | 0.08 | 0.06 |
| Impurity J | | 0.14 | 0.16 | 0.12 | 0.14 | 0.15 | 0.17 | 0.13 | 0.13 | 0.12 |
| Maximum unspecified impurities | | 0.06 | 0.05 | 0.09 | 0.11 | 0.10 | 0.08 | 0.13 | 0.26 | 0.28 |
| Total Impurities | | 0.83 | 0.94 | 0.88 | 0.99 | 1.07 | 1.44 | 1.39 | 2.07 | 2.85 |

| | | | | | | | | | | |
|---|---|---|---|---|---|---|---|---|---|---|
| Note - M: Month; ND : Not detected; RRT : Relative Retention Time | | | | | | | | | | |

### Example 7

| **Ingredients** | **Quantity per mL** | **Quantity per vial** | **Quantity (%w/v)** |
|---|---|---|---|
| Bortezomib | 2.5 mg | 12.5 mg | 0.25 % |
| Ethanol | 1.0 mg | 5 mg | 0.1 % |
| Propylene glycol | 9.0 mg | 45 mg | 0.9 % |
| Mannitol | 25 mg | 125 mg | 2.5 % |
| Water for Injection | qs to 1mL | qs to 5mL | qs to 100% |

The composition was obtained by a process similar to that in the examples above. 5ml of the composition was filled in 10ml USP Type I glass vials with rubber stoppers, each containing about 5 doses of bortezomib.

The stability testing conducted for the composition is recorded in Table 13 below -

**Table 13**

| **Condition** | **Initial** | **60°C** | **40°C±2°C/ 75±5% RH** |
|---|---|---|---|
| **Parameter** | | **1 day** | **1W** |
| Description | CCS | CCS | CCS |
| pH | 5.60 | 5.83 | 5.64 |
| Osmolality mosm/kg | 336 | 303 | 301 |
| Assay (%) | 93.7 | 92.8 | 92.9 |
| **Related substances by HPLC (%w/w)** | | | |
| Unknown Impurity @ RRT 0.24 | ND | 0.01 | 0.02 |
| Impurity C | 0.01 | 0.09 | 0.06 |
| Impurity B | 0.03 | 0.56 | 0.44 |
| Unknown Impurity @ RRT 1.24 | 0.05 | 0.21 | 0.12 |
| Impurity I | 0.08 | 0.06 | 0.18 |
| Impurity J | 0.04 | 0.11 | 0.17 |
| Maximum unspecified impurities | 0.05 | 0.05 | 0.05 |
| Total Impurities | 0.35 | 1.26 | 1.19 |

## Claims

1. A sterile, ready-to-use, aqueous composition comprising 1 to 2.5mg/ml of bortezomib, sufficient tonicity adjusting agent to provide an osmolality of from about 280 mosm/kg to about 360 mosm/kg, a solvent, a cosolvent and a pH of from about 4.5 to about 6.5, wherein the tonicity adjusting agent is mannitol, the solvent is ethanol, the cosolvent is propylene glycol, and wherein upon storage at 25°C and 60% relative humidity for six months, the bortezomib content in the composition is not less than 90%, and the amount of impurities is as follows -
a. Impurity B not more than 0.5% w/v,
b. Impurity C not more than 0.5% w/v,
c. Impurity I not more than 0.5% w/v,
d. Impurity J not more than 0.5% w/v,
e. Unknown impurities not more than 0.5% w/v, and
f. Total impurities not more than 3.5% w/v.

2. The sterile, ready-to-use, aqueous composition of claim 1 wherein the composition is free of preservatives.

3. The sterile, ready-to-use, aqueous composition of claim 1 wherein mannitol is present in an amount sufficient to provide osmolality in the range of about 280 mosm/kg to about 360 mosm/kg.

4. The sterile, ready-to-use, aqueous composition of claim 3 wherein the amount of mannitol used ranges from about 1%w/v to about 5%w/v.

5. The sterile, ready-to-use, aqueous composition of claim 1 wherein the solvent and cosolvent are together present in an amount ranging from about 0.05% w/v to about 5% w/v.

6. The sterile, ready-to-use, aqueous composition of claim 1 further comprising a stabilizer.

7. The sterile, ready-to-use, aqueous composition of claim 6 wherein the stabilizer is present in an amount ranging from about 0.01%w/v to about 2%w/v.

8. The sterile, ready-to-use, aqueous composition of claim 7 wherein the stabilizer is selected from the group consisting of arginine, alanine, glycine, methionine, lysine or proline, or a derivative or salt thereof, and mixtures thereof.

9. The sterile, ready-to-use, aqueous composition of claim 1 further comprising a chelating agent.

10. The sterile, ready-to-use, aqueous composition of claim 9 wherein the chelating agent is selected from DOTA (1, 4, 7, 10 tetraazacyclododecane - 1, 4, 7, 10 - tetraacetic acid), EDTA (Ethylenediaminetetraacetic acid), ODDA (1, 4, 10, 13 - tetraoxa - 7, 16 - diazacyclooctadecane - 7), EGTA (ethylene glycol - bis (β - aminoethyl ether) - tetraacetic acid).

11. The sterile, ready-to-use, aqueous composition of claim 10 wherein the chelating agent is present in an amount of at least 0.005%w/v.
